(19) Europäisches Patentamt
European Patent Office
Office européen des brevets

(11) Publication number: 0 013 663
B1

(12) EUROPEAN PATENT SPECIFICATION

(45) Date of publication of patent specification: 17.11.82

(21) Application number: 80810005.1

(22) Date of filing: 07.01.80

(51) Int. Cl.[3]: C 07 D 499/00, C 07 D 499/08, C 07 D 499/46, C 07 D 501/04, C 07 C 85/20, C 07 C 87/40, C 07 C 29/09, C 07 C 31/125, C 07 C 35/12 //C07D499/36

# (54) Process for removing an allylic group from allylic esters, carbonates and carbamates.

(30) Priority: 10.01.79 US 2472

(43) Date of publication of application: 23.07.80 Bulletin 80/15

(45) Publication of the grant of the patent: 17.11.82 Bulletin 82/46

(84) Designated Contracting States: BE CH DE FR GB IT NL SE

(56) References cited:

CHEMICAL COMMUNICATIONS, no. 21, 11th November 1970, pages 1392—1393 GO HATA et al.: "Palladium-catalysed exchange of allylic groups of ethers and esters with active-hydrogen compounds"

TETRAHEDRON LETTERS, no. 43, September 1970, pages 3821—3824 K.E. ATKINS et al.: "Palladium catalyzed transfer of allylic groups"

(73) Proprietor: SCHERING CORPORATION
2000 Galloping Hill Road
Kenilworth, New Jersey 07033 (US)

(72) Inventor: McCombie, Stuart Walter
159 Pleasant Valley
Way, West Orange New Jersey 07052 (US)

(74) Representative: Antony, Fritz, Dr. et al,
P.O. Box 601 Winkelriedstrasse 35
CH-6002 Lucerne (CH)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 013 663 B1

(58) References cited:
JOURNAL OF ORGANIC CHEMISTRY, vol. 38, no. 18, 7th September 1973, pages 3223—3224 E. J. COREY et al.: "Cleavage of allyloxycarbonyl protecting group from oxygen and nitrogen under mild conditions by nickel carbonyl"

JOURNAL OF THE AMERICAN CHEMICAL SOCIETY, vol. LXXII, 28th February 1950, no. 2, vol. 72, pages 725—7 C. M. STEVENS et al.: "Amino acid derivatives. I. Carboallyloxy derivatives of alpha-amino acids"

# Process for removing an allylic group from allylic esters, carbonates and carbamates

The N-allyloxycarbonyl group has been used previously as a protecting group for amines and cleaved by hydrogenation after completion of its function. In this respect, the use of allyloxycarbonyl as a protecting group offers no advantages over the more common benzyloxycarbonyl protecting group. See for instance, *J. Am. Chem. Soc.*, *72*, 725 (1950). Cleavage of allyloxycarbonyl derivatives of amines and alcohols has also been accomplished using nickel carbonyl as described in *J. Org. Chem.*, *38*, 3223 (1973). The disadvantages of this procedure are the necessary basic conditions, as well as a need for an excess of the volatile, toxic nickel carbonyl.

The use of zero-valent palladium complexes such as tetrakis-(triphenylphosphine)-palladium to catalyse the reaction of certain compounds having an active hydrogen atom with compounds having a bond between an allylic group and an oxygen atom for the purpose of synthesising compounds in which the allylic group is bonded to the residue of the active hydrogen compounds is described in *Chemical Communications. 21,* 11th November 1970 pp 1392—1393, and *Tetrahedron Letters* no. 43, September 1970 pages 3821—3824.

The present invention describes a new method for the fission of the bond between an allylic group (this meaning an unsubstituted or substituted allyl group) and an adjacent oxygen atom in an aprotic organic solvent and in the presence of an organic-soluble palladium complex having a coordinating phosphine ligand, especially by reaction with an active-hydrogen compound, characterized in that the method is applied to the removal under mild conditions of allylic groups from allylic esters or of allylic-oxycarbonyl groups from allylic carbonates or carbamates, by reacting the said allylic compounds with an alkali metal salt of a carboxylic acid having from five to eighteen carbon atoms or by using as the active-hydrogen compound the said carboxylic acid in the free form or sodium-cyanoborohydride or sodium-borohydride. This method provides near-neutral conditions which are particularly desirable for acid- or base-sensitive substrates. Thus, the so-obtainable acid, alcohol or amine is produced in high yield with a small amount of degradation by-products.

In the case of the removal of the allylic group from an allylic ester the process preferably utilizes an alkali metal salt of the carboxylic acid which is most preferably 2-ethylhexanoic acid. The reaction between the allylic ester and the alkali metal salt of e.g. 2-ethylhexanoic acid results in the formation of the alkali metal salt of the product acid which is generally insoluble in the organic, aprotic solvent utilized for the conduct of the process. As a result of the insolubility of the product salt, the reaction is driven to completion by the precipitation of the product salt. This precipitation of the product salt also results in a very pure product without the need for further recrystallizations. The removal of an allylic-oxycarbonyl group of a carbonate or carbamate to provide the corresponding alcohol or amine is advantageously accomplished utilizing the free carboxylic acid rather than the alkali metal salt thereof.

In this reaction, $CO_2$ is released as a byproduct so that the reaction is essentially irreversible.

Suitable allylic groups which are removable by the method of the present invention are any of those having the allylic structure. Typical allylic groups are those derived from allyl alcohol, haloallyl alcohol, methylallyl alcohol, crotyl alcohol, the lower alkoxycarbonyl derivatives thereof and cinnamyl alcohol or the activated esters thereof and are generally known in the art as suitable protecting groups. For simplicity and economy, allyl alcohol or haloallyl alcohol or an activated ester is usually used. Thus, particularly suitable starting materials of the method of this invention are the allylic esters, carbonates and amines wherein the allylic group or allylic-oxycarbonyl group is derived from allyl or haloallyl alcohol.

While any carboxylic acid having from five to eighteen carbon atoms or alkali metal salts thereof can be used in the method of this invention there are certain types of acids which, due to their solubility in the organic, aprotic solvent used as the reaction medium are particularly suitable. Acids having from six to ten carbon atoms and a branched chain, such as 2-propylpentanoic acid, 2-methylheptanoic acid, 2-ethylbutyric acid, 2-ethylpentanoic acid or 2-ethylheptanoic acid and the alkali metal salts thereof, are preferred. 2-Ethylhexanoic acid or an alkali metal salt thereof is most preferred due to its solubility in most aprotic solvents. Any of the alkali metal salts may be utilized but potassium and sodium are generally preferred.

The reaction proceeds at room temperature generally in a time from about 0.5 to 5 hours. The conditions are near neutral so that no other protecting or functional groups are affected during the removal reaction. Thus, the invention provides a method of selectively removing an allylic group of an allylic ester or of an allylic-oxycarbonyl group of an allylic carbonate or carbamate while leaving unaffected other protecting groups which might be present in the same molecule. The present invention may also be used to selectively deprotect one of two carboxylic acid functions in a single molecule, for instance, diallyl malonate may be converted to allyl potassium malonate.

The solvent utilized for the conduct of this invention may be any common, aprotic solvent in

which the allylic ester, carbonate or carbamate is soluble and in which the carboxylic acid or the alkali metal salt thereof or sodium-cyanoborohydride or sodium borohydride are soluble. Additionally, for ease of isolation, the product acid, (or alkali metal salt thereof) should be sparingly soluble or insoluble in the chosen solvent. Suitable solvents include dichloromethane, chloroform, carbon tetrachloride, ethyl ether, benzene, toluene, ethyl acetate, acetone, acetonitrile, tetrahydrofuran or a mixture of any of the foregoing.

The organic soluble palladium complex utilized must possess a coordinating phosphine ligand. Preferably a triarylphosphine, such as triphenylphosphine, is the coordinating phosphine ligand. The complex of choice is tetrakis (triphenylphosphine)palladium-(O). Other utilizable soluble palladium complexes are bis-(triphenylphosphine) palladium (II) dichloride, dichloro-di[benzonitrile]palladium (II) and palladium (II) diacetate in conjunction with several equivalents of triphenylphosphine (see Fieser and Fieser, "Reagents for Organic Syntheses", Vol V, pp. 497, 503, 504).

The quantity of catalyst utilized in the method of this invention is typically 0.25—5 mole percent of the allylic ester, carbonate or carbamate, with 2 mole percent being the amount most generally used. However, for large scale preparations, amounts such as 0.25 to 1 mole percent may be sufficient.

At a suitable stage prior to the removal reaction of this invention the allylic group may be introduced into any suitable molecule, for example to protect a carboxylic function, a hydroxy or an amino group, by conventional techniques. Such techniques comprise the reaction with a chloride, bromide or iodide of the allylic alcohol or an activated ester thereof. Typically, the acid or a salt thereof is reacted with the allylic bromide or iodide in a polar aprotic solvent such as dimethylformamide. The amine or alcohol is typically reacted with the chloroformate or an activated carbonate ester of the allylic alcohol in the presence of an acid acceptor. For instance, 1-aminoadamantane is reacted with allyl chloroformate or with N-[allyloxycarbonyloxy]succinimide.

The present invention is particularly useful for the removal of an allylic group or an allylic-oxycarbonyl group present in certain acid- or base-sensitive substrates. Particularly preferred substrates are beta-lactam allylic esters such as the allylic esters of penams, *e.g.*, penicillin G, ampicillin; cephems *e.g.*, cephalosporanic acid, 7 - (phenylacetamido)desacetoxycephalosporanic acid, cephamycin; carbapenems, *e.g.*, thienamycin and particularly penems. Removal of the allylic group from the foregoing affords useful antibacterial agents of commercial significance. A class of compounds for which this removal procedure has been found to be very useful are those of the formula:

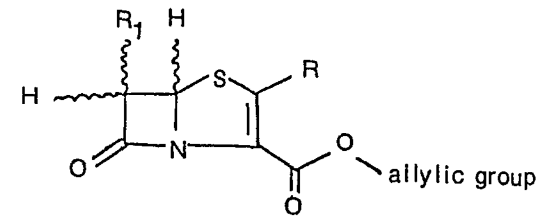

wherein R is hydrogen, lower alkyl, N-(allylic-oxycarbonyl)amino lower alkyl, or lower alkylthio the latter being optionally substituted by N-(allylic-oxycarbonyl)amino or acylamino, and $R_1$ is hydrogen, hydroxy lower alkyl or acylamino. When N-(allylic-oxycarbonyl) groups are present these may be removed during the reaction together with the allylic ester group. The free acids of the 6-unsubstituted-2-alkyl compounds of the above formula are disclosed in Current Topics in Drug Research, § IV, p. 23—25 (197). Due to the sensitivity of the penem ring structure to acidic or basic conditions, removal of the conventional protecting groups, e.g., benzyl, benzyhydryl, under standard conditions for such protecting groups causes a high percentage of degradation by-products and a low yield of the desired antibacterial 2-alkylpenem-3-carboxylic acids. See the discussion of Woodward in *Chem. Soc. Special Publications* No. 28, "Recent Advances in the Chemistry of Beta-Lactam Antibiotics", p 129 (1976). Other classes of penem antibacterial agents in which the present invention is useful are those described in U.S. Patent 4,070,477 (1978), Belgian Patents 849,118 and 866.845 and published European patent applications 636, 2210 and 3960.

Other carboxylic acids which can be produced from their corresponding allylic esters include amino acids, *e.g.,* glycine, serine, phenylalanine, *etc.;* benzoic acid and nalidixic acid.

The alcohols and amines which can be produced from their corresponding allylic carbonate or carbamate by the instant process include alcohols such as n-octadecanol, 1-menthol, 1-octanol, 2-octanol, 1-adamantanol, N-benzyloxycarbonylserine; steroids, *e.g.,* cholesterol, cortisone, testosterone, estradiol; phenol, 1-naphthol, macrolides, *e.g.,* erythromycin and rosaramicin; and amines such as 1-aminoadamantane, 2-octylamine, ephedrine, aniline, p-methoxyaniline, 1-naphthylamine, benzocaine; and amino acids, *e.g.,* glycine, phenylalanine and serine.

The following Examples illustrate the process of the invention whereas in the Preparations is shown how to obtain the starting materials.

*Preparation A*

Penicillin-G potassium salt (3.75 g) is stirred in dry dimethylformamide (12 ml) with addition of allyl iodide (1.65 g) at room temperature for 18 hours. The mixture is then diluted with ether

(100 ml), and washed with water (3 × 50 ml), 5% aqueous sodium thiosulfate (25 ml) and saturated sodium chloride (50 ml). After drying over anhydrous magnesium sulfate, the solvents are removed and the residue dried to constant weight under high vacuum to give penicillin-G allylester in near-quantitative yield, as a colorless oil with an IR spectrum in dichloromethane solution at 3350, 1785, 1730 and 1660 $cm^{-1}$.

*Preparation B*

The procedure of Preparation A is repeated with the allyl iodide being replaced by cinnamyl bromide (1.95 g). The penicillin-G cinnamyl ester is isolated in the same manner, as a white foam, with an IR spectrum in dichloromethane at 3300, 1785, 1670 and 1610 $cm^{-1}$.

*Preparation C*

The procedure of Preparation A is followed using redistilled methyl gamma-bromocrotonate (1.75 g) in place of the allyl iodide. The product, penicillin-G 3-(methoxycarbonyl)allyl ester is isolated in the same manner as a colorless, thick oil with an IR spectrum in dichloromethane at 3350, 1785, 1735, 1715 and 1680 $cm^{-1}$.

*Preparation D*

Penicillin-G allylester (10 mmol; 3.75 g) is treated at 0—5°C in dichloromethane (20 ml) with *m*-chloroperoxybenzoic acid (10.2 mmol; 1.75 g) in ethyl ether (5 ml). The solution is kept at 0—5°C for two hours, then diluted with dichloromethane (30 ml) and washed 2 × 50 ml sodium bicarbonate solution, dried and evaporated to afford a residue (foam) of the sulfoxide of penicillin-G allylester. This foam is dissolved in dry dioxane (50 ml) and refluxed for 10 hours with pyridine phosphate (0.2 g). The solvent is evaporated and the residue chromatographed on silica gel, eluting with 5% ether-dichloromethane. Evaporation of appropriate eluates gives allyl-7-phenylacetamidodesacetoxycephalosporanate, as a white foam, with an IR spectrum in dichloromethane with $\nu$ max at 3400, 1770, 1725, 1650 and 1605 $cm^{-1}$.

*Preparation E*

4-Acetylthioazetidin-2-one (Annalen, *1974*, p. 553) (1.4 g) and allyl glyoxylate hydrate (1.5 g) are refluxed for 2 hours in benzene to afford a solution of 1-(allyloxycarbonylhydroxymethyl)-4-acetylthioazetidin-2-one which is cooled and used directly in the next step.

The foregoing solution is diluted with dichloromethane (30 ml) and methanesulfonyl chloride (1.5 ml), and triethylamine (2.3 ml) is added over 2 minutes with stirring. After 5 minutes, the solution is washed with 0.2N sulfuric acid and then with water. After drying over anhydrous magnesium sulfate and evaporation, the crude 1-(allyloxycarbonylchloromethyl)-4-acetylthioazetidin-2-one is isolated as a brown oil.

The crude chloride from the previous paragraph is stirred in dry dichloromethane (15 ml) and dry dimethylformamide (15 ml) with triphenylphosphine (3,25 g) and 2,4,6-collidine (1.5 g) for 20 hours. The reaction mixture is diluted with dichloromethane and washed with 3 × 50 ml water, dried and evaporated. The residue is dried at high vacuum, then chromatographed on silica gel (100 g), eluting with dichloromethane followed by 25% ether dichloromethane. Fractions containing a mixture of the phosphorane and triphenylphosphine oxide are combined and evaporated.

The phosphorane prepared in the previous paragraph is heated at reflux for 1.5 hour in toluene (50 ml). After being cooled and evaporated, the residue is chromatographed on silica gel, eluting with 2:1 dichloromethane: hexane, followed by dichloromethane. Fractions containing the product are evaporated to give a pale yellow oil which is crystallized on strong cooling to give allyl 2-methylpenem-3-carboxylate. Infrared spectrum in dichloromethane: 1795, 1965, 1640 $cm^{-1}$.

*Preparation F*

1-Aminoadamantane (10 mmol; 1.51 g) and triethylamine (1.9 ml) in dry dichloromethane (30 ml) are stirred at 0—5°C and allyl chloroformate (1.1 ml) added dropwise. After 0.5 hour, the solution is washed with dilute sulfuric acid and water. After drying over anhydrous magnesium sulfate, the solution is evaporated to give 1-(allyloxycarbonylamino)adamantane, as a white solid, with a melting point of about 56—58°C.

Example 1

Penicillin-G allylester (0.38 g) is stirred under argon in a 0.5M dichloromethane solution of potassium 2-ethylhexanoate (3 ml) and ethyl ether (2 ml). Triphenylphosphine (0.025 g) and tetrakis (triphenylphosphine) palladium-(0) (0.020 g) are added, and stirring is continued for 1 hour at room temperature. Ethyl ether (10 ml) is added and the product filtered, washed with 1:1 ether:dichloromethane and dried *in vacuo* at 50°C to give pure potassium penicillin-G (0.34 g), identical (IR, p.m.r) with an authentic sample in a yield of about 90%.

Example 2

When the tetrakis (triphenylphosphine)palladium-(0) in Example 2 is replaced by bis (triphenylphosphine) palladium (II) dichloride (0.015 g), pure potassium penicillin-G is obtained.

Example 3

Penicillin-G cinnamyl ester (0.50 g) is stirred in ethyl ether (5 ml) and a 0.5M solution of potassium 2-ethylhexanoate (3.5 ml) in dichloromethane added. Then triphenylphosphine (0.025 g) and tetrakis (triphenylphosphine) palladium-(0) (0.22 g) are added,

and the mixture is stirred for 1 hour under argon. After dilution with ethyl ether (10 ml), the precipitate is filtered and dried to afford pure potassium penicillin-G in a yield of about 85%.

Example 4

The procedure of Example 3 is repeated with the penicillin-G cinnamyl ester being replaced by penicillin-G-(3-methoxycarbonylallyl)ester (0.46 g) to afford pure potassium penicillin-G in a yield of 79%.

Example 5

Allyl 2-methylpenem-3-carboxylate (0.10 g) is stirred in ethyl ether (1.5 ml) with a 0.5M dichloromethane solution of potassium 2-ethylhexanoate (1.25 ml). Triphenylphosphine (0.01 g) and tetrakis (triphenylphosphine)palladium-(0) (0.01 g) are added, and the mixture stirred under argon for 45 minutes at room temperature. After dilution with ethyl ether (3 ml), the product is collected, washed with ethyl ether and dried *in vacuo* at room temperature to give, as a cream solid, potassium 2-methylpenem-3-carboxylate having infrared $\nu$ max (nujol) at 1785 and 1605 $cm^{-1}$ in a yield of about 86%.

Example 6

Allyl 7-(phenylacetamido)-desacetoxycephalosporanate (1.0 g) is stirred with potassium 2-ethylhexanoate in dichloromethane (0.2M; 12 ml), triphenylphosphine (0.08 g) and bis-(triphenylphosphine)palladium (II) dichloride (0.03 g) for 1.5 hours under argon. The product is collected and washed with dichloromethane. After drying at 50°C *in vacuo*, there is afforded potassium 7 - (phenylacetamido)desacetoxycephalosporanate, identical with an authentic sample.

Example 7

1-(Allyloxycarbonylamino)adamantane (0.50 g) is stirred in dichloromethane (10 ml) containing 2-ethylhexanoic acid (0.35 g). Triphenylphosphine (0.05 g) and tetrakis(triphenylphosphine)palladium-(0) (0.04 g) are added and the mixture is stirred at room temperature for 1 hour. Ethyl ether (10 ml) is added, and the product collected, washed with ether, and dried at 25°C *in vacuo*, which is identical (IR, mp and pmr) with an authentic sample of 1-aminoadamantane prepared from the acid and amine in ether.

Example 8

N-Octadecyl allyl carbonate (0.5 g) [prepared as described in *J. Org. Chem.*, 38, 3223 (1973)] and ethylhexanoic acid (0.2 g) are stirred for 1 hour under nitrogen in dichloromethane (5 ml) containing triphenylphosphine (0.025 g) and tetrakis (triphenylphosphine)palladium-(0) (0.02 g). Thin layer chromatography indicates complete conversion to products after addition is complete. The mixture is diluted with ethyl ether, washed with sodium bicarbonate, dried and evaporated. The residue is chromatographed on silica gel and eluted with 1:1 dichloromethane hexane in order to remove the allyl 2-ethylhexanoate, and with 5% ether-dichloromethane in order to elute the product, *n*-octadecanol, fully identical with an authentic sample.

Example 9

Allyl 1-menthyl carbonate (0.3 g) [prepared as described in *J. Org. Chem.*, 38, (1973)] is substituted for the *n*-octadecyl derivative of Example 8. Cleavage is completed within 1 hour, and the product, 1-menthol, is isolated by chromatography.

Example 10

Penicillin-G 2-chloroallyl ester (1 mmol; 0.41 g) was stirred in dry tetrahydrofuran (5 ml) with sodium cyanoborohydride (2 mmol; 0.13 g) under nitrogen. Triphenylphosphine (0.05 g) and tetrakis (triphenylphosphine) palladium-(0) (0.05 g) were added and the mixture stirred under nitrogen at room temperature overnight (22 hours), by which time tlc showed only traces of ester remained. Ether (10 ml) was added, and the product collected, washed with ether and dried to give penicillin-G sodium (0.32 g) as a white powder.

Example 11

Allyl 2-ethylthiopenem-3-carboxylate (0.06 g) was stirred in dry tetrahydrofuran (1 ml) with sodium cyanoborohydride (0.02 g) triphenylphosphine (0.02 g) and tetrakis (triphenylphosphine) palladium-(0) (0.015 g) under nitrogen for 6 hours at room temperature. Ether (5 ml) was then added dropwise to precipitate the salt, which was collected by centrifuge, washed with ether and dried at room temperature to give a tan solid (0.045 g).

## Claims

1. Method for the fission of the bond between an allylic group (this meaning an unsubstituted or substituted allyl group) and an adjacent oxygen atom in an aprotic organic solvent and in the presence of an organic-soluble palladium complex having a coordinating phosphine ligand, especially by reaction with an active-hydrogen compound, characterized in that the method is applied to the removal under mild conditions of allylic groups from allylic esters or of allylic-oxycarbonyl groups from allylic carbonates or carbamates, by reacting the said allylic compounds with an alkali metal salt of a carboxylic acid having from five to eighteen carbon atoms or by using as the active-hydrogen compound the said carboxylic acid in the free form or sodium-cyanoborohydride or sodium-borohydride.

2. The method according to claim 1 wherein

a carboxylic acid having from five to eighteen carbon atoms or an alkali metal salt thereof is used.

3. The method according to claim 2 wherein the carboxylic acid has from six to ten carbon atoms and has a branched chain.

4. The method according to claim 3 wherein the carboxylic acid is 2-ethylhexanoic acid.

5. The method according to any one of claims 1 to 4 wherein the coordinating phosphine ligand of the soluble palladium complex is triphenylphosphine.

6. The method according to any one of claims 1 to 5 wherein the palladium complex is tetrakis-(triphenylphosphine)palladium-(0).

7. The method according to any one of claims 1 to 6 wherein the amount of soluble palladium complex is 0.25—5 mole percent of the allylic ester, carbonate or carbamate.

8. The method according to claim 7 wherein the amount of soluble palladium complex is 2 mole percent of the allylic ester, carbonate or carbamate.

9. Method according to any one of claims 1 to 8, when applied to the removal of allylic groups from allylic esters, characterized in that the carboxylic acid with from 5 to 18 carbon atoms is used in the form of its alkali metal salt.

10. The method according to claim 9 wherein the alkali metal salt of the carboxylic acid is the potassium or sodium salt of 2-ethylhexanoic acid.

11. The method according to any one of claims 1 to 10 wherein the allylic ester is a beta-lactam allylic ester.

12. The method according to claim 11 wherein the beta-lactam allylic ester is selected from a penam, cephalosporin, carbapenem, and penem allylic ester.

13. The method according to claim 12 wherein the penem allylic ester is of the formula

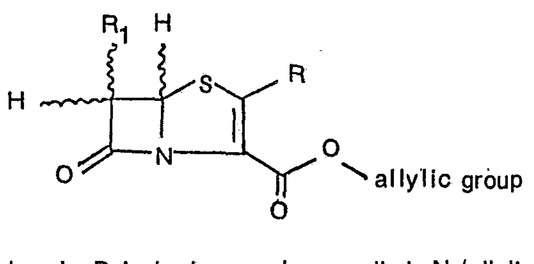

wherein R is hydrogen, lower alkyl, N-(allylic-oxycarbonyl)amino lower alkyl, or lower alkylthio the latter being optionally substituted by N-(allylic-oxycarbonyl)amino or acylamino, and $R_1$ is hydrogen, hydroxyloweralkyl or acylamino; and wherein, during the reaction, both, the allylic ester group and the N-(allylic-oxycarbonyl)-group, are removed.

**Revendications**

1. Procédé pour la fission de la liaison entre un groupe allyle (cela signifiant un groupe allyle non substitué ou substitué) et un atome d'oxygène adjacent dans un solvant organique aprotique et en présence d'un complexe de palladium soluble dans les produits organiques ayant un ligand de phosphine de coordination, en particulier par réaction avec un composé d'hydrogène actif, caractérisé en ce que le procédé est appliqué à l'enlèvement, en conditions modérées, des groupes allyles, d'esters allyliques ou de groupes allyloxycarbonyles, de carbonates ou carbamates d'allyle, par réaction desdits composés allyliques avec un sel d'un métal alcalin d'un acide carboxylique ayant de 5 à 18 atomes de carbone ou en utilisant, comme composé d'hydrogène actif, ledit acide carboxylique sous forme libre ou cyanoborohydrure de sodium ou borohydrure de sodium.

2. Procédé selon la revendication 1, où l'on utilise un acide carboxylique ayant de 5 à 18 atomes de carbone ou son sel d'un métal alcalin.

3. Procédé selon la revendication 2, où l'acide carboxylique a de 6 à 10 atomes de carbone et a une chaîne ramifiée.

4. Procédé selon la revendication 3, où l'acide carboxylique est l'acide 2-éthylhexanoïque.

5. Procédé selon l'une des revendications 1 à 4, où le ligand de phosphine de coordination du complexe de palladium soluble est de la triphénylphosphine.

6. Procédé selon l'une des revendications 1 à 5, où le complexe de palladium est du tétrakis-(triphénylphosphine)palladium-(0).

7. Procédé selon l'une des revendications 1 à 6, où la quantité du complexe de palladium soluble est de 0.25 à 5 moles% de l'ester, carbonate ou carbamate d'allyle.

8. Procédé selon la revendication 7, où la quantité du complexe de palladium soluble est de 2 moles% de l'ester, carbonate ou carbamate d'allyle.

9. Procédé selon l'une des revendications 1 à 8, appliqué à l'enlèvement des groupes allyles d'esters allyliques, caractérisé en ce qu'on utilise un acide carboxylique ayant de 5 à 18 atomes de carbone, sous forme de son sel d'un métal alcalin.

10. Procédé selon la revendication 9, où le sel d'un métal alcalin de l'acide carboxylique est le sel de potassium ou de sodium de l'acide 2-éthylhexanoïque.

11. Procédé selon l'une des revendications 1 à 10, où l'allyl ester est un allyl ester de béta-lactame.

12. Procédé selon la revendication 11, où l'allyl ester de béta-lactame est choisi parmi un péname, de la céphalosporine, un carbapénem et un allyl ester de pénem.

13. Procédé selon la revendication 12, où l'allyl ester de pénem a pour formule

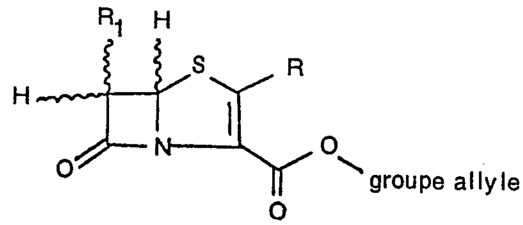

où R est de l'hydrogène, un alcoyle inférieur, un N-(allyloxycarbonyl)amino alcoyle inférieur, ou un alcoylthio inférieur, ce dernier étant éventuellement substitué par un N-(allyloxycarbonyl)amino ou acylamino et $R_1$ est de l'hydrogène, un hydroxyalcoyle inférieur ou un acylamino; et où, pendant la réaction, le groupe allyl ester et le groupe N-(allyloxycarbonyle) sont retirés.

**Patentansprüche**

1. Verfahren zur Spaltung der Bindung zwischen einer Allyl-Gruppe (hier: einer unsubstituierten oder substituierten Allyl-Gruppe) und einem benachbarten Sauerstoff-Atom in einem aprotischen organischen Lösungsmittel und in Gegenwart eines organisch-löslichen Palladium-Komplexes mit einem koordinativ gebundenen Phosphin-Liganden insbesondere durch Reaktion mit einer Verbindung mit aktivem Wasserstoff, dadurch gekennzeichnet, daß das Verfahren unter milden Bedingungen zur Entfernung von Allyl-Gruppen aus Allylestern oder von Allyloxycarbonyl-Gruppen aus Allylcarbonaten oder -carbamaten angewandt wird, indem diese Allyl-Verbindungen mit einem Alkalimetall-Salz einer Carbonsäure mit fünf bis achtzehn Kohlenstoff-Atomen umgesetzt werden oder indem die betreffende Carbonsäure in freier Form oder Natriumcyanoborhydrid oder Natriumborhydrid als Verbindung mit aktivem Wasserstoff verwendet werden.

2. Verfahren nach Anspruch 1, dadurch gekennzeichnet, daß eine Carbonsäure mit fünf bis achtzehn Kohlenstoff-Atomen oder eines ihrer Alkali-Salze verwendet wird.

3. Verfahren nach Anspruch 2, dadurch gekennzeichnet, daß die Carbonsäure sechs bis zehn Kohlenstoff-Atome und eine verzweigte Kette besitzt.

4. Verfahren nach Anspruch 3, dadurch gekennzeichnet, daß die Carbonsäure 2-Ethylhexansäure ist.

5. Verfahren nach Anspruch 1 bis 4, dadurch gekennzeichnet, daß der koordinativ gebundene Phosphin-Ligand des löslichen Palladium-Komplexes Triphenylphosphin ist.

6. Verfahren nach Anspruch 1 bis 5, dadurch gekennzeichnet, daß der Palladium-Komplex Tetrakis-(triphenylphosphin)palladium (0) ist.

7. Verfahren nach Anspruch 1 bis 6, dadurch gekennzeichnet, daß die Menge des löslichen Palladium-Komplexes 0,25 bis 5 Molprozent des Allylesters, -carbonats oder -carbamats beträgt.

8. Verfahren nach Anspruch 7, dadurch gekennzeichnet, daß die Menge des löslichen Palladium-Komplexes 2 Molprozent des Allylesters, -carbonats oder -carbamats beträgt.

9. Verfahren nach Anspruch 1 bis 8, dadurch gekennzeichnet, daß bei Anwendung zur Entfernung von Allyl-Gruppen aus Allylestern die Carbonsäure mit 5 bis 18 Kohlenstoff-Atomen in Form ihres Alkali-Salzes eingesetzt wird.

10. Verfahren nach Anspruch 9, dadurch gekennzeichnet, daß das Alkalimetall-Salz der Carbonsäure das Kalium- oder Natrium-Salz von 2-Ethylhexansäure ist.

11. Verfahren nach Anspruch 1 bis 10, dadurch gekennzeichnet, daß der Allylester ein beta-Lactam-allylester ist.

12. Verfahren nach Anspruch 11, dadurch gekennzeichnet, daß der beta-Lactam-allylester ausgewählt ist aus einem Penam-, Cephalosporin-, Carbapenem- und Penem-allylester.

13. Verfahren nach Anspruch 12, dadurch gekennzeichnet, daß der Penem-allylester einer der Formel

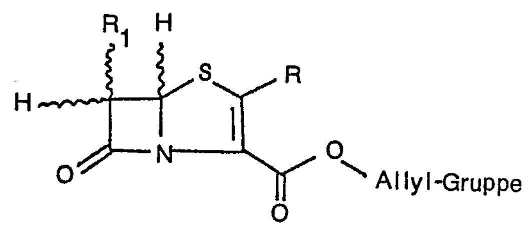

ist, in der

R Wasserstoff, niederes Alkyl, N-(Allyloxycarbonyl)amino-niederes Alkyl oder niederes Alkylthio, letzteres gegebenfalls substituiert durch N-(Allyloxycarbonyl)amino oder Acylamino, ist und

$R^1$ Wasserstoff, Hydroxy-niederes Alkyl oder Acylamino ist,

und im Laufe der Reaktion sowohl die Allylester-Gruppe als auch die N-(Allyloxycarbonyl)-Gruppe entfernt werden.